# EUROPEAN PATENT SPECIFICATION

(11) **EP 1 962 940 B1**
(45) Date of publication and mention of the grant of the patent: **23.10.2019**
(21) Application number: 06846655.6
(22) Date of filing: 18.12.2006
(51) Int. Cl.: A61M 25/10, A61M 25/01

(54) **BALLOON CATHETER WITH CENTRALIZED VENT HOLE**
BALLONKATHETER MIT ZENTRALISIERTEM LUFTLOCH
CATHETER A BALLONNET AVEC TROU DE VENTILATION CENTRALISE

(30) Priority: 23.12.2005 US 752878 P
(43) Date of publication of application: 03.09.2008
(73) Proprietor: C.R. Bard, Inc., Murray Hills, NJ 07974 (US)
(72) Inventor: STAPLETON, Corey, E., Gilbert, AZ 85296 (US)
(74) Representative: Hoffmann Eitle
(86) International application number: PCT/US2006/062232
(87) International publication number: WO 2007/076324

(56) References cited:
- WO-A1-96/39999
- FR-A1- 2 716 114
- US-A- 4 877 031
- US-A- 4 930 341
- US-A- 5 611 775
- US-A1- 2002 082 548
- US-A1- 2005 234 499
- US-B1- 6 203 558
- US-B1- 6 306 162
- US-B1- 6 503 223
- US-B1- 6 648 854
- US-B1- 6 648 854

## Description

The present invention relates generally to balloon catheter assemblies for use in angioplasty and stent delivery procedures. In particular, the present invention provides a system for delivery of a balloon catheter to a stenosed blood vessel and inflation of the dilation balloon to expand a stent implant and/or the stenosed blood vessel.

### Background Art

A large number of balloon catheters have been devised for angioplasty and stent delivery procedures. For example, US-A-4877031 discloses a balloon catheter useful in angioplasty procedures. Commonly a guide wire is first introduced percutaneously into the patient's vascular system, advanced and then steered to the site of a stenosis. A dilation balloon or catheter is then advanced over the guide wire until the balloon is positioned within the stenosis so that on inflation, the balloon will compress the stenosis by dilatation of the blood vessel to thereby re-establish a more adequate blood flow path past the stenosis. To facilitate even compression pressure distribution along the length of the stenosed lesion, it is preferred that the dilation balloon be centered relative to the stenosis so as to fully engage the lesion.

Balloon dilation catheters have also been utilized in stetn delivery in which the stseant is disposed about the balloon and inflated into place at athe stenosis. Catheter operators seek accurate deployment of the stent directly on the diseased tissue of the vessel in order to avoid stent migration to eiter side of the diseased tissue threby avoiding or minimizing the chance of leaving some of the diseased tissue untreated. Accurate stent deployment is also desirable in order to avoid adversely affecting healthy tissue.

Stent misplacements may occur because of specific inflation dynamics experienced by the expandable balloon when deploying the stent. Known stent delivery catheters inflate the balloon portion of the catheter preferentially from either the distal or proximal end of the balloon. During inflation, the expanding balloon may form an unsymmetrical growth or inflation wave that may be said to drive or plow the stent so that it opens progressively from one end to the other along the front of the inflation wave. The wave may sometimes cause the stent to disengage prematurely from the balloon. This form of balloon inflation is referred to as "end-to end" preferential inflation. End-to-end balloon inflation may further cause a deploying stent to displace longitudinally away from its intended delivery site, thereby potentially ineffectively treating the diseased lesion within the patient's vasculature.

Known balloon dilation catheters used in connection with stent deployment and/or other applications are shown and described in several U.S. Patents including: U.S. Patent Nos. 6,136,011; 5,908,448; 5,226,880; 5,176,619; 4,811,737; 5,409,495; 5,334,148; 5,169,386; and 3,939,820. In U.S. Patent No. 6,592,568, described is one inflation technique for medial inflation of the balloon using an intermediate balloon inside a stent delivering dilation balloon to concentrate a bolus of fluid medially for distribution through the dilation balloon. The intermediate balloon can either be rupturable or otherwise provide a controlled fluid leak to release fluid into the dilation balloon. This technique, however, adds complexity to the procedure by requiring controlled bursting or leakage of an intermediate balloon.

Another complex stent delivery and deployment device is shown and described in U.S. Patent No. 6,203,558 in which a stent is disposed about an inflation balloon. The inflation balloon is disposed about a catheter assembly having an inner shaft and an outer shaft. The inflation balloon is inflated from its proximal end by the delivery of a pressurized fluid flowing between the inner and outer shafts. The deployment device also includes an expandable securement device disposed about the inner shaft and disposed within the inflation balloon. The inner shaft has a single lumen for carrying a guide wire and fluid for expanding the securement device. To expand the securement member, fluid is discharged from the single lumen through a valve disposed along the inner shaft and centrally located within the securement member. For example, see FIG. 34 of the '558 Patent. The expanded securement member secures the engagement between the inflation balloon and the stent.

We are directed to make reference to US-A-5611775, which discloses a catheter with an inflatable member at its distal end, and is used for delivering a diagnostic fluid. See also US-A-4930341 which discloses a balloon protector sleeve used in testing the balloon for leaks.

Another patent, U.S.Patent No. 6,648,854, also discloses a single lumen balloon tipped catheter for inflating a balloon having an operating pressure of about one atmosphere. The catheter effectively utilizes a single lumen to carry both a guide wire and inflation fluid. However, where balloons having higher operating pressures are utilized, a single lumen device may not be sufficient to provide the adequate pressure for inflating the balloon.

US 4,877,031 teaches a balloon dilation catheter for vascular procedures which provides for the perfusion of blood when the balloon is inflated to dilate the artery or otherwise impede blood flow. The catheter comprises an elongated tubular member having an inner lumen extending along the length thereof, a perfusion body secured to the distal end of the tubular member having a small diameter lumen in fluid communication with the inner lumen of the tubular member, and a large diameter lumen which allows for the perfusion of blood through the perfusion body, and a balloon disposed about the perfusion body and secured around the periphery thereof in at least two circular locations. An inflation/deflation port connects the small diameter lumen in the perfusion body to the interior of the balloon to pass inflation fluid there between. A guide wire is disposed within the small diameter inner lumen of the tubular member and facilitates the advancement of the catheter across the stenosis. When the balloon is inflated, blood proximal to the balloon passes through the large diameter lumen and discharges distally thereto.

US 6,203,558 discloses a system for delivery and deployment of an inflation expandable stent within a vessel, comprising a catheter having a proximal and distal end; a stent, inflation expandable from a delivery diameter to a deployment diameter, such that the delivery diameter is reduced from the deployment diameter for conforming the stent to the catheter, such that the stent, in its delivery diameter, is coaxially mounted on the catheter near the catheter distal end; an expandable inflation member coaxially mounted on the catheter axially within the stent, for expansion of the stent from the delivery diameter to the deployment diameter upon application of fluid deployment pressure to the inflation member; and a securement component coaxially mounted on the catheter, axially within the expandable inflation members, the securement component designed and adapted to provide a securement pressure to the stent in the delivery diameter to maintain the stent in position on the catheter during delivery to the deployment site.

WO 9639999 discloses an intraluminal grafting system which comprises a generally sinusoidal wire frame having apices which extend longitudinally outward from the end of the tubular body apices which are secured within the tubular body. Both the protruding apices and the base apices are formed with helices which bias the attachment system radially outward. The attachment system further includes a plurality of lumen piercing members that are oriented in a responsive relationship to the radially outward bias of the attachment system. The graft may include a plurality of synthetic fiber tufts secured to the outer surface of the tubular body to facilitate sealing the graft within the vessel. The graft may also include a plurality of crimps formed in the tubular body of the graft.

US 6,306,162 discloses a stent delivery system which incorporates a polyurethane balloon which exhibits a high coefficient of friction with respect to metallic substrates. Through the process of stent expansion, the delivery system creates a differential in the rate of axial growth of metallic stents. The differential in growth causes a center portion of the stent to experience greater axial expansion than respective end portions. The lower axial expansion of the end portions results in a stent having end portions of comparatively higher radial stiffness than the corresponding center portion of the stent.

### Disclosure of Invention

The invention is defined in the independent claim below. The dependent claims are directed to preferred embodiments and optional features. The present invention provides a catheter assembly for engaging a stenosis and for stent delivery. The assembly includes a catheter including a wall having a proximal end and a distal end along a longitudinal axis. The wall has an interior surface and an exterior surface, in which the interior surface defines a first lumen and a second lumen spaced apart and disposed about the longitudinal axis. The wall defines an opening extending between the interior surface and the exterior surface. The opening is in communication with the first lumen to define a flow path having an angle incident to the longitudinal axis. The exterior surface further includes a first marker; and a second marker spaced apart from one another along the longitudinal axis so as to be substantially equidistant from the opening. The assembly also includes a balloon having a first end and a second end defining a holding volume therebetween. The first end and the second ends are sealed about the exterior surface. The opening is disposed within the holding volume thereby placing the first lumen in sealed fluid communication with the holding volume for inflating and deflating the balloon through the opening. The first and second ends of the balloon are further spaced substantially equidistantly about the opening along the longitudinal axis. A balloon dilatable stent is disposed about the balloon. The balloon is configured to engage the stent with a stenosis and effect stent delivery by channeling a contrast fluid along the catheter and into the holding volume of the balloon through the opening to fully dilate the balloon and the stent. The balloon has a non-tapered central region and two tapered ends. The stent is disposed about the non-tapered central region of the balloon only, the stent being evenly and radially expanded about the central region of the balloon.

Applicant recognizes that it is desirable to have an apparatus for centrally locating the dilation balloon catheter assembly within a stenosed region to ensure proper engagement between the stenosis and the dilation balloon. The catheter assembly is combined with a stent to form a stenosis treatment device, the stent being disposed about the balloon to engage the stent with a stenosis. It is desirable to have an apparatus for medial inflation of a dilation balloon to evenly expand the stent. Preferably, proper medial inflation and location of the dilation balloon in the stenosed region forms a "dog bone" shape. The "dog bone" shape results as the stenosis compresses evenly on the central portion of the dilated balloon and/or stent. This balloon inflation dynamic can limit stent migration along the balloon and thereby minimize any misplacement in stent deployment. Accordingly, it is desirable to provide for consistent medial inflation of the dilation balloon such that the balloon expands evenly and radially from a central point, thus avoiding uneven distortions in the dilation balloon as it is inflated.

The first marker and the second matter are disposed within the holding volume. In addition, at least one of the first marker and the second marker are radiopaque and/or radiographic. Moreover, the exterior surface of the wall of the catheter defines a first diameter outside the holding volume and a second diameter inside the holding volume. Preferably, the second diameter is smaller than the first diameter and the catheter includes a taper portion between the first and second diameter.

A fluid delivery device not forming part of the present invention is also described. The fluid delivery device includes an elongated member having a proximal end and a distal end defining a first lumen and a second lumen spaced apart along a longitudinal axis. The first lumen is configured to convey a fluid, and the member has an opening disposed between the proximal and distal ends in fluid communication with the lumen. The delivery device further includes a first radiopaque and/or radiographic marker and a second radiopaque and/or radiographic marker. The first marker and the second marker are disposed along the longitudinal axis and spaced from one another so as to be substantially equidistant from the opening.

The present invention is useful in an example not forming part of the present invention of a method of engaging a stenosis with an inflatable member having a first end and a second end in which the inflatable member has disposed therein at least a portion of a tubular member having a first radiopaque and/or radiographic marker and a second radiopaque and/or radiographic marker spaced along a longitudinal axis of the tubular member. The method includes locating the first and second markers equidistantly about a portion of the stenosis such that the inflatable member is substantially centered along the length of the portion of the stenosis. The method further includes: flowing a fluid in a channel of the tubular member along the longitudinal axis and introducing a sufficient amount of the fluid into the inflatable member through an opening of the tubular member to expand the inflatable member substantially radially and engage the stenosis. A stent is disposed about the inflatable member such that introducing a sufficient amount of fluid into the inflatable member further engages the stent with the stenosis.

Dilating a stenosis
can be achieved by locating a first marker of a catheter assembly to one side of a portion of a stenosis and locating a second marker on the opposite side of the portion such that the first and second markers are generally equidistant from the portion of the stenosis. The method further includes disposing the fluid fill opening of an inflatable member generally equidistant between the first and second markers, and expanding the inflatable member via the fluid fill opening substantially equally longitudinally and radially about the central region to engage and apply an expansion force to the portion.

### Brief Description of the Drawings

The accompanying drawings
illustrate a preferred embodiment of the invention, and together with the general description given above and the detailed description given below, serve to explain the features of the invention.
FIG. 1 is an illustrative perspective view of an embodiment of a balloon catheter assembly.
FIG. 1A is an isometric view of the proximal end of the assembly of FIG. 1.
FIG. 1B is a geometric plan view of the assembly of FIG. 1.
FIG. 2 is a detailed portion of the distal end of the assembly of FIG. 1.
FIG. 2A is a detailed portion of the assembly of FIG. 2.
FIG. 3 is a cross-sectional detail of the assembly of FIG. 2.
FIG. 3A is perspective view of a portion of the assembly of FIG. 2.
FIG. 4 is an illustrative example of the assembly of FIG. 1 used in a stenosis treatment procedure.

### Mode(s) For Carrying Out the Invention

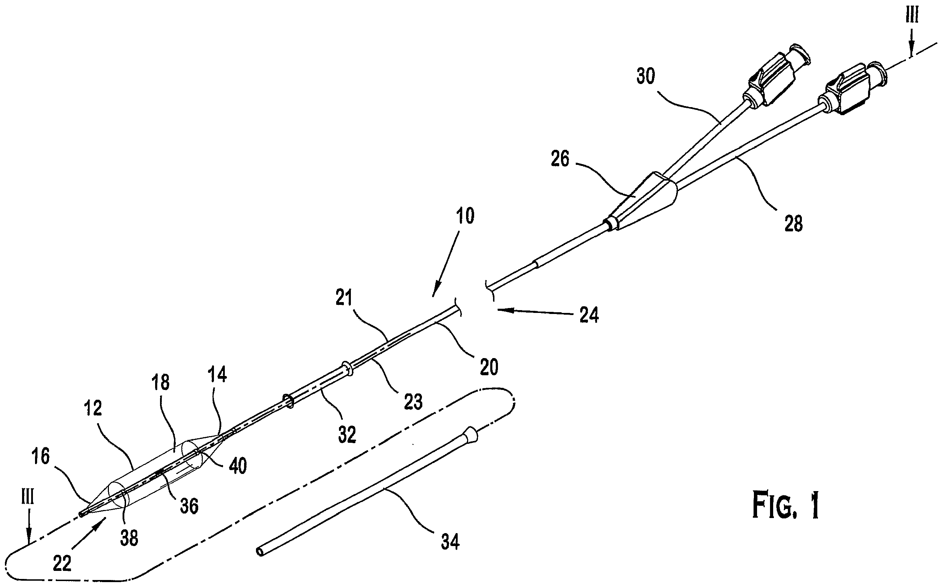

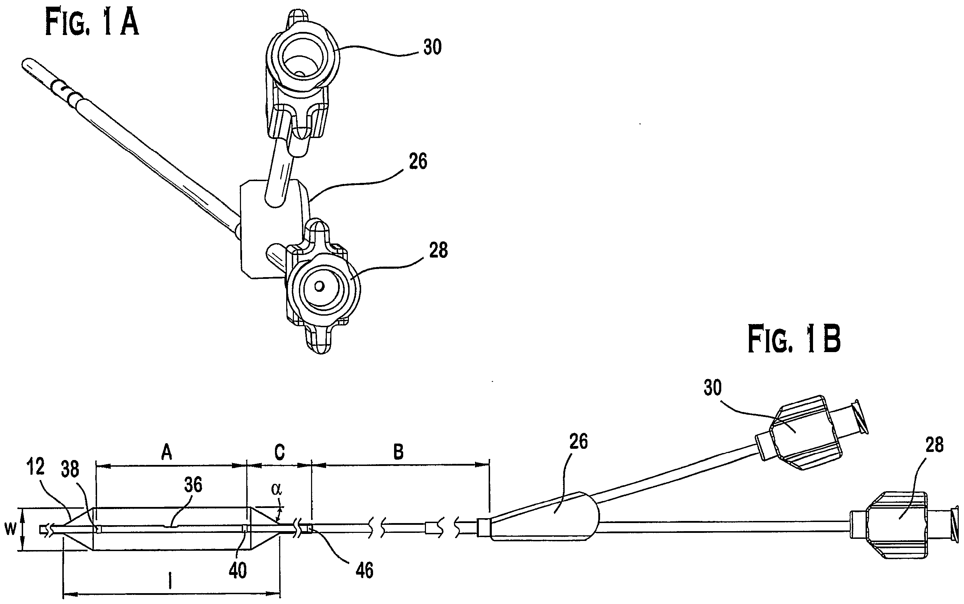

FIG. 1 shows a preferred embodiment of a catheter assembly 10 for engaging a stenosis. More specifically, the catheter assembly 10 can be configured for angioplasty procedures in which an inflatable member or balloon 12 is introduced into a blood vessel for engagement with a diseased portion of the blood vessel such as, for example, a stenosis and for engagement with an implantable prosthesis such as, for example, a stent or stent-graft. The catheter assembly 10 is configured for introducing an implant or stent (not shown) into the blood vessel to treat the stenosis. The stent is disposed about the balloon 12 and the catheter assembly 10 can deliver and position the stent in engagement with the stenosis for implantation.

The catheter assembly 10 can subsequently engage the stent at the stenosis site and inflate the balloon 12 to expand the stent for engagement with the stenosis.

Generally, the catheter assembly 10 includes a catheter 20 having a proximal portion 24 a distal portion 22. The catheter 20 preferably is an elongated tubular member having a wall 21 forming a exterior surface 23 and an interior 25 surface (not shown) defining a longitudinal axis III--III. The catheter 20 is preferably formed by extrusion of a thermoplastic material such as, for example, PEBAX 7300® thermoplastic material with a gel content of 9 percent or less compounded with 10 percent Bismuth Subcarbonate. Preferably disposed at the proximal portion 24 is a connector 26 having a first port 28 for introducing a guide wire into the catheter 20 and a second port 30 for introducing a fluid. Disposed at the distal portion 22 of the catheter 20 is the dilation balloon 12. The dilation balloon 12 is disposed about the distal portion 22 of the catheter 20 so as to locate an opening 36 in the catheter 20 within the holding volume 18 of the balloon 12. Fluid is exchanged between the balloon 12 and the catheter 20 through the opening 36 to inflate and deflate the balloon 12. To assist an operator in locating the balloon 12 along a stenosis or other targeted region, the catheter 20 includes first and second, preferably radiographic and/or radiopaque, markers 38, 40 along the distal portion 22 inside the holding volume 18 of the balloon 12.

The balloon 12 of catheter assembly 10 has a first end 14, a second end 16 to define the holding volume 18 therebetween. The first and second ends 14, 16 are disposed about the catheter 20. The first end 14 and second end 16 of the balloon 12 are sealed about the catheter 20 so as to enclose a distal portion 22 of the catheter 20 within the holding volume 18 in a fluid-tight manner. For example, the first and second ends 14, 16 can be thermally bonded to the exterior surface 23 of the catheter 20 to form a fluid-tight seal. Alternative bonding techniques can be used to seal the ends 14 and 16 to the catheter 20 such as, for example, laser or adhesive bonding techniques. In addition, the balloon 12 can be coupled to the catheter 20 in any other manner to enclose the distal portion 22 of the catheter 20 within the holding volume 18 in a fluid-tight manner. The balloon 12 is preferably constructed from a nylon material, such as, Nylon 12 or Nylon 11, or alternatively from other suitable thermoplastic polymers such as, for example, polyether block amide (PEBA), polyethylene, polyethylene terephthalate (PET). Moreover, the balloon can be a composite material balloon formed from a combination of Nylon and other polymers or a combination of ultra high molecular weight polyethylene by itself or with PET. The balloon 12 defines a sufficient strength in an inflated state so as to dilate or expand a stent.

One technique for forming the balloon 12 includes blow molding a Nylon or PET tube under heat in a mold to form the desired shape, for example, a circular cylindrical body with two conical tapered ends. The formed balloon 12 can be disposed over and thermally bonded to the catheter 20. U.S. Patent No. 5,755,690 describes one method for forming a multiple layer high strength balloon for dilation catheter in which a parison, of orientable semicrystalline polymer such as, for example PET, is disposed within a mold with one end of the parison sealed and the other end secured to a fluid source such as, for example, a gas. The parison is axially drawn and radially expanded within the mold to form an expanded balloon. The expanded balloon can then be exposed to a heat step in order to increase crystallinity in the balloon for dimensional stability. The balloon can then be removed from the mold and disposed about the catheter and thermally bonded thereto. Alternatively to thermally bonding the balloon 12, an adhesive can be employed to bond the balloon 12 to the catheter 20.

The distal portion 22 and the proximal portion 24 of the catheter 20 are preferably formed as a unitary construction joined together by a transition section 46. Alternatively, the distal portion 22 and the proximal portion 24 can be distinct elements mechanically joined together by the transition 46. Preferably, the outer diameter of the proximal portion 24 is larger than the outer diameter of the distal portion 22 of the catheter 20. The transition section 46 is preferably tapered from the proximal portion 24 to the distal portion 22. Alternatively, transition section 46 can have a constant diameter to join the proximal portion 24 to the distal portion 22 thereby forming a step transition from the proximal portion 24 to the distal portion 22.

The connector 26 disposed at the proximal end 24 of the catheter 20 can be coupled to the catheter 20 by any suitable techniques such as, for example, interference fit, thread connection or press fit. The connector 26 is preferably disposed proximal of the balloon 12. The connector 26 is configured for introducing a fluid, guide wire or any other instrumentation into the catheter 20. Specifically, the connector 26 includes a first port 28 configured for receipt of a guide wire (not shown) to be inserted along the vein or artery of the patient The catheter assembly 10 can be disposed about the guide wire so that an operator can guide the assembly 10 along the wire to locate the assembly to a desired location relative to the stenosis within the vein or artery. More specifically and preferably, the balloon 12 can be generally centered across the stenosed lesion. The first port 28 is preferably aligned parallel to or coaxial with the longitudinal axis III--III of the catheter 20.

The connector 26 includes a second port 30 configured to connect to a fluid source (not shown). The fluid source can be, for example, a syringe or other pump/vacuum device for delivery of a fluid. The fluid is preferably a liquid and can be, for example, a dye, a saline solution or any other contrast fluid to inflate the balloon 12. Shown in FIG. 1A is another embodiment of the connector 26. The second port 30 can be configured for receipt of a syringe as a fluid source to inject and withdraw fluid through the assembly 10. The second port 30 of FIG. 1 is preferably in fluid communication with the first port 28 within the connector 26, however the connector 26 can be configured so as to isolate the fluids from the second port 30 with the first port 28. The port 30 can form an angle incident with the catheter 20. The port 30 forms an acute angle incident to the longitudinal axis III--III of the catheter 20 in the direction of fluid flow moving distally away from an operator. During a procedure, the fluid is
introduced into the second port 30 and further into the catheter 20. The fluid is discharged from an opening 36 in the distal portion 22 of the catheter 20 and into the holding volume 18 to expand the balloon 12. Preferably, the fluid is introduced into the balloon 12 to expand the balloon radially from the opening 36, along and about the longitudinal axis III--III. The port 30 can also be used to extract fluid from and deflate the balloon 12. Fluid can be drawn from the balloon 12 into the catheter 20 through the opening 36 and returned to the fluid source via the connector 26 and port 30.

FIG. 1 and FIG. 1B show the balloon 12 in an inflated state with FIG. 1B providing particular geometric relationships of the assembly 10. In the inflated state, the balloon 12 is shown as a substantially tubular or cylindrical member along the longitudinal axis III--III. In a plane perpendicular to the longitudinal axis III--III, the balloon 12 defines a cross-sectional section that is preferably circular, however other cross-sections are possible such as, for example, oval, multi-lobed or other polygons. The width w (preferably the diameter) of the balloon 12, as seen in FIG. 1B, can range from about 1 millimeter to about 40 millimeters, preferably range from about 1 millimeter to about 26 millimeters and even more preferably range from about 3 millimeters to about 20 millimeters, and the length *l* of the balloon 12 can range from about 10 millimeters to about 120 millimeters. Each end of the balloon 12 is preferably conical so as to preferably defines a cone angle α relative to a line parallel to the longitudinal axis III--III. The cone angle can range from about five degrees (5°) to about thirty degrees (30°) depending upon the length *l* of the balloon. The dimensions A, B and C of the catheter 20 can vary along with the width w and length I of the balloon 12. More specifically, dimension A measured from the first preferably radiopaque and/or radiographic marker 38 to the second preferably radiopaque and/or radiographic marker 40 can be of any suitable length and preferably any one of about, 10 millimeters, 15 millimeters, 20 millimeters, 30 millimeters, 40 millimeters, 60 millimeters, 80 millimeters, 100 millimeters, to about 120 millimeters in length. Dimension B, measured from the transition section 46 to the connector 26 can preferably be of any suitable length and preferably, any one of about, 40 centimeters, 75 centimeters, 115 centimeters, 130 centimeters, to about 140 centimeters in length. Dimension C measured from the transition section 46 to the second marker 40 can preferably be any one of about, 10 millimeters, 15 millimeters, to about 20 millimeters in length.

Referring again to FIG. 1, the catheter assembly 10 can also include a deflator 32 that is preferably a sliding member 32 disposed about the outer surface 23 of the catheter 20. The sliding member 32 can be permitted to slide along the catheter 20 between the distal and proximal portions 22, 24. The sliding member 32 can be configured to assist in deflating the balloon member 18 by passing over the balloon 12 to displace any fluid and/or air in the holding volume 18. The sliding member 32 can include a central channel through which the balloon 12 and the catheter 20 can pass. The body of the sliding member 32 is preferably substantially spool shaped to provide a low profile and easy handling for the operator; however, other geometries are possible permitting manual manipulation. The catheter assembly 10 can also include a removable cap 34. The cap 34 can engage and disengage from the balloon 12 and the distal portion 22 of catheter 22 to protect the balloon 12 from damage when not in use.

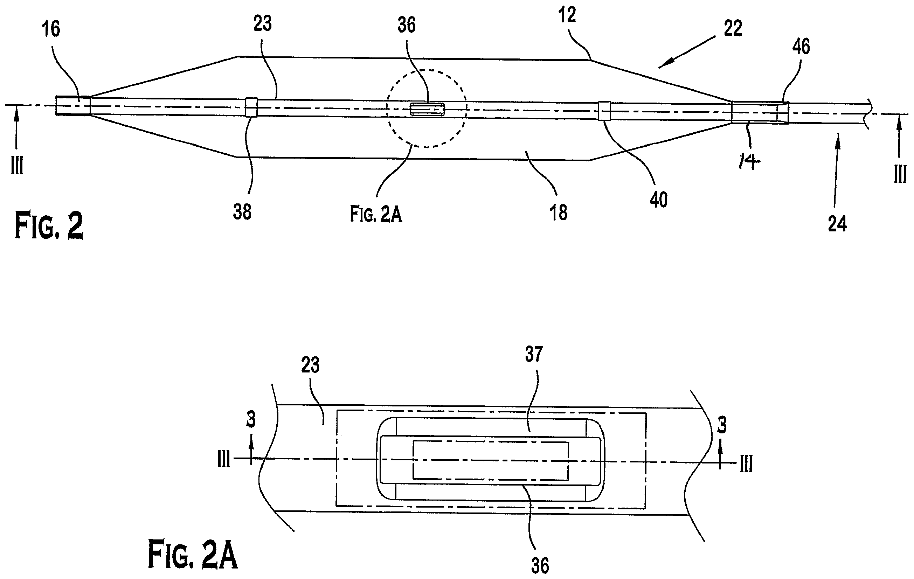

FIG. 2 shows an enlarged view of the distal portion 22 of the catheter 20 sealed within the balloon 12. The distal portion 22 of the catheter 20 further includes the opening 36. Preferably, first and second ends 14, 16 of the balloon 12 are secured about the catheter 20 so as to be equidistantly spaced from the opening 36 and thus place the opening 36 in a substantially central location within the holding volume 18 of the balloon 12. Fluid introduced into the catheter 20 is discharged through the opening 36 to inflate the balloon 12 from an initial deflated state or volume (not shown) to a substantially inflated state or volume (as shown in FIG. 2).

Shown in FIG. 2A is the plan view detail of the opening 36. The opening 36 is preferably rectangular and elongated in the direction of the longitudinal axis III--III so as to deliver and evacuate a sufficient volume of fluid to respectively inflate and deflate the balloon 12. The opening 36 can further include a chamfer or transition 37 from the interior of the catheter 20 to the outer surface 23, and the edges of the opening 36 along the outer surface 23 are preferably rounded to assist in achieving the desired flow characteristics. Where, for example, the opening 36 is rectangular, the dimensions of opening 36 can measure about 0.2 centimeters in length and about 0.02 centimeters in width. Generally, opening 36 can have any dimensioned geometry and transition characteristics such as, for example, a substantially circular, oval or polygonal, so long as the desired flow characteristics are obtained for the rapid inflation and deflation of the balloon 12. Preferably the opening 36 is dimensioned and configured in a manner that provides for the inflation and deflation of the balloon 12 within a time period that minimizes the time for which the blood vessel may be occluded by the balloon 12. As described above, the dimensions of the catheter 20 can vary with the dimensions of the balloon 12. Accordingly, the dimensions of the opening 36 and the balloon 12 can be such as to define a relationship over various configurations of the catheter 20. Specifically, in one preferred embodiment, the area of the opening 36 and the fully expanded holding volume 18 of the balloon 12 can define a ratio of area to volume. This ratio can be constant over the various configurations of the catheter 20. Alternatively, the ratio of the area of the opening 36 and the fully expanded holding volume 18 of the balloon 12 can be variable over the various configurations of the catheter 20.

The centralized location of the opening 36 shown in FIG. 2 relative to the balloon 1 2 provides a fluid distribution within the balloon 12 to facilitate the even and radial expansion of the balloon 12 from the deflated state to the inflated state. More specifically, the fluid discharging from the substantially central point within the holding volume 18 of the balloon 12 engages interior surfaces of the balloon equally radially and evenly along the direction of the longitudinal axis III--III. Thus, uneven concentrations of fluid or waves which can distort the shape of the balloon 12 are minimized or otherwise avoided. This can ensure that a target area (e.g., stenosis or stent) is engaged fully and evenly by the balloon 12 or stent to produce the preferable "dog bone" shape the balloon 12. In a case where the balloon 12 is being used to implant a stent, the centralized expansion of the balloon 12 ensures that the stent is expanded evenly along its length.

The distal portion 22 of the catheter 20 further includes the first marker 38 and the second marker 40 disposed on the exterior surface 23 of the catheter 20. Preferably, the markers 38, 40 are made of a radiopaque and/or radiographic material such as, for example, 18 Karat Gold, platinum, tantalum, BaSO₄, Iridium to make the catheter 20 or at least the distal portion 22 visible under fluoroscopic observation. The markers 38, 40 can be used by an operator to guide the catheter assembly 10 under fluoroscopic observation to a desired location within the blood vessel. The first and second radiopaque and/or radiographic markers 38, 40 are spaced apart and located along the longitudinal axis III--III such that the markers are equidistantly spaced from the opening 36. The markers 38, 40 are disposed within the holding volume 18. Because the first and second ends 14, 16 of the balloon 12 are also centered about the opening 36, the first and second markers 38, 40 can facilitate the centering of the balloon 12 with respect to the target area. In particular, a clinician can utilize the radiopaque markers 38, 40 under fluoroscopic observation to center the opening 36 along the length of the target area, such as a stenosed lesion, and because of the fixed relation of the balloon ends 14, 16 to the opening 36, the balloon is thereby preferably centered with respect to the target region for properly engaging the length of the target region.

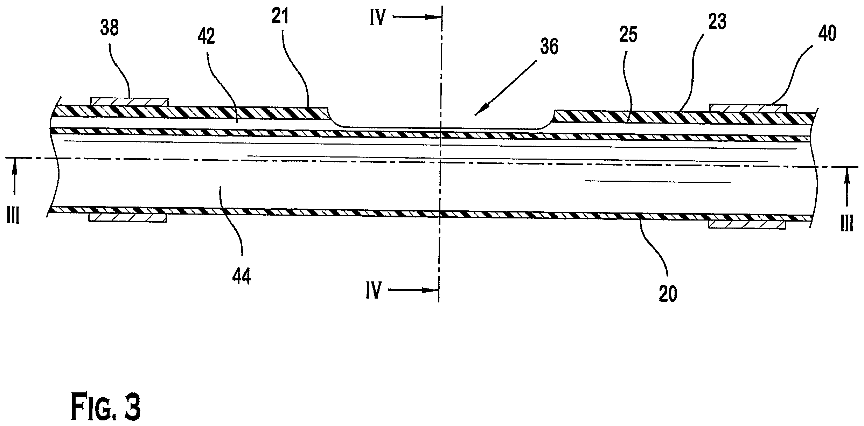

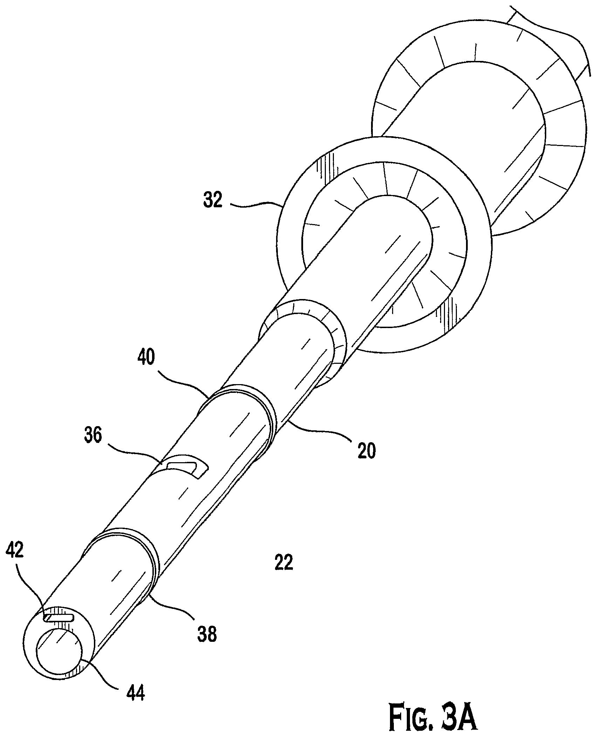

Shown in FIG. 3 is a cross-sectional view of a portion of the distal portion 22 of the catheter 20. The interior surface 25 of the wall 21 forming the catheter 20 can further define a first channel or lumen 42, preferably parallel to the longitudinal axis III--III. The lumen 42 can extend from the distal portion 22 into the proximal portion 24 of catheter 20 for communication with the second port 30 of the connector 26 in order to exchange a fluid, preferably a liquid, between the balloon 12 and the fluid source for inflation/deflation of the balloon 12. The inner diameter of the lumen 42 is dimensioned to provide a sufficient flow of fluid given the delivery pressures from the fluid source such as, for example, a syringe. The inner diameter of the first lumen 42 can remain constant over the entire length of the catheter 20 or alternatively, the inner diameter of the first lumen 22 can change over the length of the catheter 20. The lumen 42 is preferably offset from the centerline longitudinal axis III--III of the catheter 20.

To facilitate fluid exchange between the balloon 12 and the catheter 20, the lumen 42 is in fluid communication with the holding volume 18 via the opening 36 shown in FIGS. 2 and 3. More specifically, the opening 36 is positioned relative to the lumen 42 so as to define a fluid path having an angle incident to the longitudinal axis III--III. Fluid conveyed along the lumen 42 can be discharged from the opening 36 and into the holding volume 18 to expand the balloon 12. Preferably, the flow path is substantially orthogonal to the longitudinal axis III--III to radially disperse the fluid from a substantially central portion of the holding volume 18. Alternatively, the opening 36 can be positioned and configured so as to define a fluid path having an acute angle with longitudinal axis III--III so long as the fluid path can be dispersed from a substantially central portion of the holding volume 18.

Shown in FIG. 3A is an end view of the catheter 20. Preferably, the cross-section of the first lumen 42 is substantially rectangular and more preferably is crescent shape to convey an adequate flow of fluid to and from the holding volume 18. The first lumen 42 can be dimensioned and configured so as to adequately fit within the overall size constraints of the catheter 20 such as, for example, the outer diameter of the catheter 20 and the demands on cross-sectional area of the catheter 20 to accommodate any additional lumen. The cross-sectional area of the lumen 42 can define other geometries such as substantially circular, for example, so long as the lumen 42 is dimensioned to convey the adequate fluid flow. In a preferred embodiment, the lumen 42 is sealed at the distal end so as to provide a sufficient discharge pressure at the opening 36 to promote the even radial expansion of the balloon 12. Generally, the balloon 12 is rated for an operational pressure ranging from about 405,3 kPa (4 atmosphere (atm.)) to about 810.6 kPa (8 atmosphere (atm.)) and is more preferably about 810.6 kPa 8 atm.), which corresponds to an operational delivery pressure of about 861.84 kPa (125 psi). Depending on the size of the balloon 12, the balloon 12 can further be configured for rated burst pressures ranging from about 810.6 kPa (8 atm.) to about 1621.2 kPa (16 atm.). Alternatively, the lumen 42 can have multiple discharge openings so long as a sufficient discharge pressure is provided at the opening 36.

Fluid in the holding volume 18 can be drawn through the opening 36 and into the lumen 42 to deflate the balloon 12. In addition to facilitating the radial expansion of the balloon 12, the central positioning of the opening 36 relative to the holding volume 18 can maximize the time for which the opening 36 remains patent as fluid is drawn through the opening 36 and the balloon 12 collapses about the distal end 22 of the catheter 20 and eventually over opening 36. Accordingly, the positioning of the opening 36 can control the efficiency of deflation of the balloon 12. The efficiency of balloon deflation can define the time required to deflate the balloon 12 thereby defining the period that an inflated balloon 12 blocks or restricts the flow of blood through the blood vessel. Generally, it is desired that the time period for which the expansion of balloon 12 blocks blood flow through the blood vessel be minimized.

The catheter 20 shown in FIG. 3 preferably includes a second channel or lumen 44 distinctly defined by the wall 21 extending parallel to the longitudinal axis III--III and the first lumen 42. The second lumen 44 is dimensioned and configured to receive a guide wire upon which the catheter assembly 10 can translate. The second lumen 44 separates the guide wire from the fluid flow in the lumen 42, thereby eliminating interference with the flow or pressure characteristics of the fluid by the presence of the guide wire. Preferably, the second lumen 44 extends from the distal end to the proximal end of the catheter 20 for communication with the first port 28 of the connector 26. The second lumen 44 is preferably dimensioned and configured to receive the guide wire from the port 28. The guide wire can be a conventional surgical guide wire such as, for example, stainless steel type 302 or 304 having an outer diameter of about 0.25 millimeter. The first and second lumen 42, 44 can alternatively be defined by distinct tube members within a single larger catheter tube (not shown).

The inner diameter of the second lumen 44 can remain constant over the entire length of the catheter 20 or alternatively, the inner diameter of the second lumen 44 can change over the length of the catheter 20 to accommodate space demands on the overall cross-sectional area of the catheter 20. Preferably, the overall cross-sectional area of the catheter 20 remains constant over the various configurations of the catheter 20 discussed above. Alternatively, the overall cross-sectional area of the catheter 20 can vary proportionally with any one or more of the dimensions defining the catheter 20 such as, for example, the catheter's overall length or the lengths A, B or C described above. Shown in FIG. 3A is the cross-section of the lumen 44 as being substantially circular to provide the guide wire a substantially smooth wall through which to pass. Alternatively, other geometries are possible such as rectangular, oval or any other configuration so long as the lumen 44 is dimensioned to permit passage of the guide wire.

The second lumen 44 is preferably offset from the centerline longitudinal axis III--III of the catheter 20 to accommodate the dimension and configuration of the first lumen 42 for the delivery of the proper operating pressure for inflating the balloon 12. The catheter can be dimensioned to accommodate additional lumen to provide channels for the insertion of other fluids or devices such as, for example, a third lumen to carry a temperature probe (not shown).

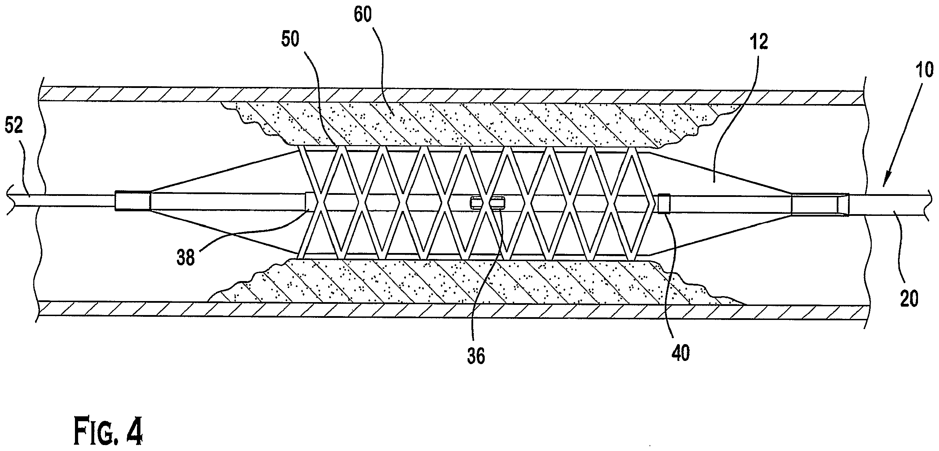

Shown in FIG. 4 is an illustrative depiction of a stent delivery procedure in which the preferred embodiment of the catheter assembly 10 described above is locating and positioning a stent 50 along a stenosis 60 for expansion of the stenosed lesion and blood vessel 62. The catheter assembly 10 is preferably disposed about a guide wire 52, and an operator using the assembly 10 under fluoroscopy observation can align the balloon 10 and the stent 50 with the stenosis and further identify a portion of the stenosis 60 to which a direct expansion force using the balloon 12 of the assembly 10 can be applied. Preferably, the identified portion is the central portion of the stenosis 60. Accordingly, the operator slides the catheter assembly 10 along the guide wire 52 to align the radiopaque markers 38, 40 equidistantly about the central portion of the stenosis 60 and thereby align a substantially central region of the balloon 12 with the central portion of the stenosis.

A contrast fluid can be channeled along the catheter 20 and introduced into the holding volume 18 of the balloon 12 through the opening 36 to fully dilate the balloon 12 and the stent 50 as shown. The preferably fixed centralized relation of the opening 36 to the markers 38, 40 aligns the opening 36 with the identified portion of the stenosis to be expanded, and with the opening 36 being preferably centrally located in the holding volume 18, the balloon 12 and stent 50 are evenly and radially expanded about the central region of the balloon 12 into engagement with the stenosis to apply expansion forces at least to the identified portion.

The various configurations of the catheter assembly 10 described herein provide numerous advantages in the performing angioplasty and stent delivery procedures. The catheter 20 preferably includes two spaced apart lumen for separately carrying a guide wire and an inflation fluid. The separately dedicated lumen can facilitate delivery of the inflation fluid at the proper operating pressure to expand the inflation balloon 12 by minimizing or eliminating interference of the guide wire with the fluid flow or delivery pressure. The opening 36 of catheter 20 is preferably disposed centrally within the holding volume 18 to facilitate central and localized fluid delivery within the holding volume 18 to promote even radial expansion of the balloon 12. The even radial expansion of the balloon 12 can ensure proper engagement between the balloon 12 and the stent or stent graft so as to evenly radially expand the stent device and prevent migration of the stent device along the balloon 12. In addition, the centralized location of the opening 36 relative to the holding volume 18 can increase the efficiency of the balloon deflation by maximizing the patency of the opening 36 to withdraw fluid from the balloon 12 while minimizing the time balloon remains in an expanded state to occlude the blood vessel being treated. In addition, the markers 38, 40 are preferably located within the holding volume 18 and relative to the opening 36 of the catheter 20 to provide the necessary visual indicators to center the balloon 12 relative to the target area or region. The radiopaque and/or radiographic markers 38, 40 assist in properly locating the balloon and/or stent or stent graft relative to the center of a target region or center.

While the present invention has been disclosed with reference to certain embodiments, numerous modifications, alterations and changes to the described embodiments are possible without departing from the scope of the claims below. The present invention has the full scope defined by the following claims. As used herein, the singular form of "a", "an" and "the" include the plural unless specifically defined as only one.

## Claims

1. A catheter assembly (10) for engaging a stenosis and for stent delivery, the assembly comprising:
a catheter (20) including a wall (21) having a proximal end (24) and a distal end (22) disposed along a longitudinal axis, the wall having an interior surface (25) and an exterior surface (23), the interior surface defining a first lumen (42) and a second lumen (44) spaced apart and disposed about the longitudinal axis, the wall defining an opening (36) extending between the inner surface and the exterior surface, the opening being in communication with the first lumen so as to define a flow path having an angle incident to the longitudinal axis;
a first marker (38) disposed about the exterior surface along the longitudinal axis; and
a second marker (40) disposed about the exterior surface along the longitudinal axis, the first and second markers being spaced from one another along the longitudinal axis so as to be substantially equidistant from the opening;
a balloon (12) having a first end (14) and a second end (16) defining a holding volume (18) therebetween, the first end and the second ends being sealed about the exterior surface, the opening being disposed within the holding volume so as to place the first lumen in sealed fluid communication with the holding volume for inflating and deflating the balloon through the opening, the first and second ends being spaced substantially equidistantly about the opening along the longitudinal axis; and the balloon having a non-tapered central region and two tapered ends; and
a balloon dilatable stent (50) disposed about the balloon, the balloon being configured to engage the stent with a stenosis and effect stent delivery by chaneling a contrast fluid along the catheter (20) and into the holding volume (18) of the balloon (12) through the opening (36) to fully dilate the balloon (12) and the stent (50), and **characterised in that**:
the stent is disposed about the non-tapered central region of the balloon only, the stent being evenly and radially expanded about the central region of the balloon.

2. The catheter assembly of claim 1, wherein the holding volume defines a first volume and a second volume larger than the first volume, the opening being configured so as to alter the holding volume between the first and the second volumes substantially radially about the opening.

3. The catheter assembly of claim 2, wherein the balloon has an operational pressure of about 810.6 kPa (8 atm.) so as to define a fluid delivery pressure at the opening to alter the holding volume between the first and the second volumes.

4. The catheter assembly of claim of any one of the above claims, wherein the balloon has a width ranging from 1 millimeter to 40 millimeters.

5. The catheter assembly of any one of the above claims, wherein the balloon has a length ranging from 10 millimeters to 120 millimeters.

6. The catheter assembly of any one of the above claims, further comprising a connector disposed about the exterior surface proximate the balloon, the connector having a first port and a second port in communication with the second lumen, the first port being in fluid communication with the first and the second lumen.

7. The catheter assembly of claim 6, wherein the first port defines an angle incident to the first lumen and the second port is substantially coaxial with the second lumen.

8. The catheter assembly of any one of the above claims, wherein the first marker and the second marker are disposed within the holding volume.

9. The catheter assembly of any one of the above claims, wherein at least one of the first marker and the second marker comprises at least one of a radiopaque and radiographic material.

10. The catheter assembly of any one of the above claims, wherein the exterior surface of the wall defines a first diameter outside the holding volume, the exterior surface defining a second diameter inside the holding volume, the second diameter being smaller than the first diameter, the exterior surface including a taper portion between the first and the second diameter.

11. The catheter assembly of any one of the above claims, further comprising a deflator disposed about the exterior surface, the deflator having a first position proximate the balloon and a second position distal the balloon, the deflator being configured to translate along the longitudinal axis from the first position to the second position so as to alter the holding volume.

12. The catheter assembly of any one of the above claims, further comprising a cap engaged with the catheter such that the balloon is disposed inside the cap.

13. The catheter assembly of any one of the above claims, wherein the first lumen defines a substantially crescent shaped cross-sectional area perpendicular to the longitudinal axis.

14. The catheter assembly of any one of the above claims, wherein the first lumen defines a substantially rectangular cross-sectional area perpendicular to the longitudinal axis.

15. The catheter assembly of claim 13 or 14, wherein the opening comprises a substantially rectangular opening.

16. The catheter assembly of claim 13, 14 or 15, wherein the second lumen is a guidewire lumen.

17. The catheter assembly of claim 13, 14, 15 or 16, further comprising a connector (26) disposed about the exterior, the connector having a first port (28) in communication with the first lumen and a second port (30) in communication with the second lumen.

## Patentansprüche

1. Katheterbaugruppe (10) zum Eingreifen in eine Stenose und zur Stentzuführung, wobei die Baugruppe Folgendes umfasst:
einen Katheter (20), beinhaltend eine Wand (21), aufweisend ein proximales Ende (24) und ein distales Ende (22), angeordnet entlang einer Längsachse, wobei die Wand eine innere Oberfläche (25) und eine äußere Oberfläche (23) aufweist, wobei die innere Oberfläche ein erstes Lumen (42) und ein zweites Lumen (44) definiert, die beabstandet und um die Längsachse herum angeordnet sind, wobei die Wand eine Öffnung (36) definiert, die sich zwischen der inneren Oberfläche und der äußeren Oberfläche erstreckt, wobei die Öffnung mit dem ersten Lumen in Verbindung steht, um einen Strömungsweg, der einen zu der Längsachse inzidenten Winkel aufweist, zu definieren;
einen ersten Marker (38), der um die äußere Oberfläche herum entlang der Längsachse angeordnet ist; und
einen zweiten Marker (40), der um die äußere Oberfläche herum entlang der Längsachse angeordnet ist, wobei die ersten und zweiten Marker voneinander entlang der Längsachse beabstandet sind, um im Wesentlichen äquidistant zu der Öffnung zu sein;
einen Ballon (12), der ein erstes Ende (14) und ein zweites Ende (16) aufweist, die ein Haltevolumen (18) dazwischen definieren, wobei das erste Ende und die zweiten Enden um die äußere Oberfläche herum abgedichtet sind, wobei die Öffnung innerhalb des Haltevolumens angeordnet ist, um das erste Lumen zum Inflatieren und Deflatieren des Ballons durch die Öffnung in abgedichteter Fluidverbindung mit dem Haltevolumen zu platzieren, wobei die ersten und zweiten Enden im Wesentlichen äquidistant um die Öffnung herum entlang der Längsachse beabstandet sind; und der Ballon eine nichtkonische zentrale Region und zwei konische Enden aufweist; und
einen ballondilatierbaren Stent (50), der um den Ballon herum angeordnet ist, wobei der Ballon konfiguriert ist, um den Stent in eine Stenose eingreifen zu lassen und die Stentzuführung durch Schleusen eines Kontrastfluids entlang des Katheters (20) und in das Haltevolumen (18) des Ballons (12) durch die Öffnung (36) zu bewirken, um den Ballon (12) und den Stent (50) vollständig zu dilatieren, und **dadurch gekennzeichnet, dass**:
der Stent nur um die nichtkonische zentrale Region des Ballons herum angeordnet ist, wobei der Stent gleichmäßig und radial um die zentrale Region des Ballons herum ausgedehnt ist.

2. Katheterbaugruppe nach Anspruch 1, wobei das Haltevolumen ein erstes Volumen und ein zweites Volumen, das größer ist als das erste Volumen, definiert, wobei die Öffnung konfiguriert ist, um das Haltevolumen zwischen den ersten und den zweiten Volumen im Wesentlichen radial um die Öffnung herum zu verändern.

3. Katheterbaugruppe nach Anspruch 2, wobei der Ballon einen Betriebsdruck von etwa 810,6 kPa (8 atm.) aufweist, um einen Fluidzuführdruck an der Öffnung zu definieren, um das Haltevolumen zwischen den ersten und den zweiten Volumen zu verändern.

4. Katheterbaugruppe nach Anspruch nach einem der vorstehenden Ansprüche, wobei der Ballon eine Weite im Bereich von 1 Millimeter bis 40 Millimeter aufweist.

5. Katheterbaugruppe nach einem der vorstehenden Ansprüche, wobei der Ballon eine Länge im Bereich von 10 Millimeter bis 120 Millimeter aufweist.

6. Katheterbaugruppe nach einem der vorstehenden Ansprüche, weiter umfassend ein Anschlussstück, das um die äußere Oberfläche herum benachbart zu dem Ballon angeordnet ist, wobei das Anschlussstück einen ersten Port und einen zweiten, mit dem zweiten Lumen in Verbindung stehenden Port aufweist, wobei der erste Port mit dem ersten und dem zweiten Lumen in Fluidverbindung steht.

7. Katheterbaugruppe nach Anspruch 6, wobei der erste Port einen zu dem ersten Lumen inzidenten Winkel definiert und der zweite Port im Wesentlichen koaxial zu dem zweiten Lumen ist.

8. Katheterbaugruppe nach einem der vorstehenden Ansprüche, wobei der erste Marker und der zweite Marker innerhalb des Haltevolumens angeordnet sind.

9. Katheterbaugruppe nach einem der vorstehenden Ansprüche, wobei mindestens einer des ersten Markers und des zweiten Markers mindestens eines von einem röntgendichten und radiografischen Material umfasst.

10. Katheterbaugruppe nach einem der vorstehenden Ansprüche, wobei die äußere Oberfläche der Wand einen ersten Durchmesser außerhalb des Haltevolumens definiert, wobei die äußere Oberfläche einen zweiten Durchmesser innerhalb des Haltevolumens definiert, wobei der zweite Durchmesser kleiner ist als der erste Durchmesser, wobei die äußere Oberfläche einen Konusabschnitt zwischen dem ersten und dem zweiten Durchmesser beinhaltet.

11. Katheterbaugruppe nach einem der vorstehenden Ansprüche, weiter umfassend einen Deflator, der um die äußere Oberfläche herum angeordnet ist, wobei der Deflator eine erste Position benachbart zu dem Ballon und eine zweite Position distal zu dem Ballon aufweist, wobei der Deflator konfiguriert ist, um sich entlang der Längsachse von der ersten Position zu der zweiten Position zu verschieben, um das Haltevolumen zu verändern.

12. Katheterbaugruppe nach einem der vorstehenden Ansprüche, weiter umfassend eine Kappe, die so in den Katheter eingreift, dass der Ballon innerhalb der Kappe angeordnet ist.

13. Katheterbaugruppe nach einem der vorstehenden Ansprüche, wobei das erste Lumen eine im Wesentlichen halbmondförmige Querschnittsfläche senkrecht zu der Längsachse definiert.

14. Katheterbaugruppe nach einem der vorstehenden Ansprüche, wobei das erste Lumen eine im Wesentlichen rechteckige Querschnittsfläche senkrecht zu der Längsachse definiert.

15. Katheterbaugruppe nach Anspruch 13 oder 14, wobei die Öffnung eine im Wesentlichen rechteckige Öffnung umfasst.

16. Katheterbaugruppe nach Anspruch 13, 14 oder 15, wobei das zweite Lumen ein Führungsdrahtlumen ist.

17. Katheterbaugruppe nach Anspruch 13, 14, 15 oder 16, weiter umfassend ein Anschlussstück (26), das um das Äußere herum angeordnet ist, wobei das Anschlussstück einen ersten Port (28) in Verbindung mit dem ersten Lumen und einen zweiten Port (30) in Verbindung mit dem zweiten Lumen aufweist.

## Revendications

1. Ensemble de cathéter (10) pour s'engager sur une sténose et fournir un stent, l'ensemble comprenant :
un cathéter (20) incluant une paroi (21) ayant une extrémité proximale (24) et une extrémité distale (22) disposées le long d'un axe longitudinal, la paroi ayant une surface interne (25) et une surface externe (23), la surface interne définissant une première lumière (42) et une seconde lumière (44) espacées l'une de l'autre et disposées autour de l'axe longitudinal, la paroi définissant une ouverture (36) s'étendant entre la surface interne et la surface externe, l'ouverture étant en communication avec la première lumière de manière à définir un trajet d'écoulement ayant un angle incident sur l'axe longitudinal ;
un premier marqueur (38) disposé autour de la surface externe le long de l'axe longitudinal ; et
un second marqueur (40) disposé autour de la surface externe le long de l'axe longitudinal, les premier et second marqueurs étant espacés l'un de l'autre le long de l'axe longitudinal de manière à être sensiblement équidistants de l'ouverture ;
un ballonnet (12) ayant une première extrémité (14) et une seconde extrémité (16) définissant entre elles un volume de maintien (18), la première extrémité et la seconde extrémités étant étanchées autour de la surface externe, l'ouverture étant disposée dans le volume de maintien de manière à placer la première lumière en communication fluidique étanche avec le volume de maintien pour gonfler et dégonfler le ballonnet à travers l'ouverture, les première et seconde extrémités étant espacées de manière sensiblement équidistante autour de l'ouverture le long de l'axe longitudinal ; et le ballonnet ayant une région centrale non amincie et deux extrémités amincies ; et
un stent dilatable (50) disposé autour du ballonnet, le ballonnet étant configuré pour s'engager sur le stent avec une sténose et effectuer la fourniture du stent en canalisant un fluide de contraste le long du cathéter (20) et dans le volume de maintien (18) du ballonnet (12) à travers l'ouverture (36) de manière à dilater complètement le ballonnet (12) et le stent (50), et **caractérisé en ce que** :
le stent est disposé autour de la région centrale non amincie du ballonnet seulement, le stent étant déployé de manière uniforme et radiale autour de la région centrale du ballonnet.

2. Ensemble de cathéter selon la revendication 1, dans lequel le volume de maintien définit un premier volume et un second volume plus grand que le premier volume, l'ouverture étant configurée pour modifier le volume de maintien entre le premier et le second volumes sensiblement radialement autour de l'ouverture.

3. Ensemble de cathéter selon la revendication 2, dans lequel le ballonnet a une pression opérationnelle d'environ 810,6 kPa (8 atm.) de manière à définir une pression de fourniture de fluide à l'ouverture pour modifier le volume de maintien entre le premier et le second volumes.

4. Ensemble de cathéter selon la revendication selon l'une quelconque des revendications précédentes, dans lequel le ballonnet a une largeur allant de 1 millimètre à 40 millimètres.

5. Ensemble de cathéter selon l'une quelconque des revendications précédentes, dans lequel le ballonnet a une longueur allant de 10 millimètres à 120 millimètres.

6. Ensemble de cathéter selon l'une quelconque des revendications précédentes, comprenant en outre un raccord disposé autour de la surface externe proche du ballonnet, le raccord ayant un premier orifice et un second orifice en communication avec la seconde lumière, le premier orifice étant en communication fluidique avec la première et la seconde lumière.

7. Ensemble de cathéter selon la revendication 6, dans lequel le premier orifice définit un angle incident sur la première lumière et le second orifice est sensiblement coaxial avec la seconde lumière.

8. Ensemble de cathéter selon l'une quelconque des revendications précédentes, dans lequel le premier marqueur et le second marqueur sont disposés à l'intérieur du volume de maintien.

9. Ensemble de cathéter selon l'une quelconque des revendications précédentes, dans lequel au moins l'un du premier marqueur et du second marqueur comprend au moins l'un d'un matériau radio-opaque et d'un matériau radiographique.

10. Ensemble de cathéter selon l'une quelconque des revendications précédentes, dans lequel la surface externe de la paroi définit un premier diamètre à l'extérieur du volume de maintien, la surface externe définissant un second diamètre à l'intérieur du volume de maintien, le second diamètre étant plus petit que le premier diamètre, la surface externe incluant une partie qui s'amincit entre le premier et le second diamètre.

11. Ensemble de cathéter selon l'une quelconque des revendications précédentes, comprenant en outre un dispositif de dégonflement disposé autour de la surface externe, le dispositif de dégonflement ayant une première position proche du ballonnet et une seconde position distale vis-à-vis du ballonnet, le dispositif de dégonflement étant configuré pour effectuer une translation le long de l'axe longitudinal de la première position à la seconde position de manière à modifier le volume de maintien.

12. Ensemble de cathéter selon l'une quelconque des revendications précédentes, comprenant en outre une coiffe en prise avec le cathéter de sorte que le ballonnet soit disposé à l'intérieur de la coiffe.

13. Ensemble de cathéter selon l'une quelconque des revendications précédentes, dans lequel la première lumière définit une surface en coupe transversale sensiblement conformée en croissant perpendiculairement à l'axe longitudinal.

14. Ensemble de cathéter selon l'une quelconque des revendications précédentes, dans lequel la première lumière définit une surface en coupe transversale de forme sensiblement rectangulaire perpendiculairement à l'axe longitudinal.

15. Ensemble de cathéter selon la revendication 13 ou 14, dans lequel l'ouverture comprend une ouverture sensiblement rectangulaire.

16. Ensemble de cathéter selon la revendication 13, 14 ou 15, dans lequel la seconde lumière est une lumière guide-fil.

17. Ensemble de cathéter selon la revendication 13, 14, 15 ou 16, comprenant en outre un raccord (26) disposé à l'extérieur, le raccord ayant un premier orifice (28) en communication avec la première lumière et un second orifice (30) en communication avec la seconde lumière.
